# EUROPÄISCHE PATENTSCHRIFT

(11) **EP 0 931 069 B1**
(45) Veröffentlichungstag und Bekanntmachung des Hinweises auf die Patenterteilung: **29.11.2000**
(21) Anmeldenummer: 97918992.5
(22) Anmeldetag: 21.08.1997
(51) Int. Cl.: C07D 213/78, C07D 271/06, C07D 401/12, C07D 413/12, C07B 43/00

(54) **VERFAHREN ZUR HERSTELLUNG VON AMIDINEN**
PROCESS FOR PREPARING AMIDINES
PROCEDE DE PRODUCTION D'AMIDINES

(30) Priorität: 03.09.1996 DE 19635674
(43) Veröffentlichungstag der Anmeldung: 28.07.1999
(73) Patentinhaber: BASF AKTIENGESELLSCHAFT, 67056 Ludwigshafen (DE)
(72) Erfinder: SCHÄFER, Bernd, D-76889 Dierbach (DE); ZIERKE, Thomas, D-67459 Böhl-Iggelheim (DE)
(86) Internationale Anmeldenummer: EP9704548
(87) Internationale Veröffentlichungsnummer: WO9809950

(56) Entgegenhaltungen:
- H. MOSER ET AL.: "Poly(dipeptamidinium)-Salze: Definition und Methoden zur präparativen Herstellung" HELVETICA CHIMICA ACTA., Bd. 69, 1986, BASEL CH, Seiten 1224-1262, XP002049723 in der Anmeldung erwähnt
- B.J. GOUR-SALIN ET AL.: "Inhibition of papain by peptide nitriles: conversion of the nitrile group into other functionalities via the papain:nitrile thioimidate ester adduct." CANADIAN JOURNAL OF CHEMISTRY., Bd. 69, Nr. 8, 1991, OTTAWA CA, Seiten 1288-1297, XP002049724
- JENDRALLA H ET AL: "EFFICIENT KG-SCALE SYNTHESIS OF THROMBIN INHIBITOR CRC 220" TETRAHEDRON, Bd. 51, Nr. 44, 30.Oktober 1995, Seiten 12047-12068, XP000644579 in der Anmeldung erwähnt
- P.C. SRIVASTAVA ET AL.: "Synthesis and biological activity of nucleotides related to the antitumor agent 2-beta-D-ribofuranosylthiazole-4-carboxami de" JOURNAL OF MEDICINAL CHEMISTRY., Bd. 27, 1984, WASHINGTON US, Seiten 266-269, XP002049725 in der Anmeldung erwähnt

## Beschreibung

Die vorliegende Erfindung betrifft ein neues Verfahren zur Herstellung von Amidinen.

Amidine lassen sich auf vielfältige Weise herstellen. Hierunter ist die Pinner-Reaktion gefolgt von einer Ammonolyse des Iminocarbonsäureesters eine der bewährtesten Methoden (Ber. 18 (1885) 2845). Von Nachteil ist hierbei die zweistufige Reaktionsführung. In der Regel benutzt man große Überschüsse an Chlorwasserstoff, die letztlich zu einer großen Salzfracht führen und gelegentliche Abtrennprobleme bereiten. Schließlich sind bei dieser Reaktionssequenz die Reaktionszeiten verhältnismäßig lang und die Umsätze und Ausbeuten nur mittelmäßig. In analoger Weise zur Pinner-Reaktion können auch Mercaptane als Hilfsreagenzien zur Herstellung von Amidinen eingesetzt werden (R.C. Schnur, J. Org. Chem. 44 (1979) 3726). Eine Synthesevariante hiervon ist die Addition von Schwefelwasserstoff an Nitrile zu Thiocarbonsäureamiden, gefolgt von einer Alkylierung des Schwefels und der Ammonolyse (H. Rappoport, J. Org. Chem. 46 (1981) 2455; M. Ohno, Tetrahedron Lett. (1979) 2517). In allen Fällen hat man es hierbei mit sehr übel riechenden und hochtoxischen Verbindungen zu tun. Zur Alkylierung benutzt man in der Regel Methyliodid oder Dimethylsulfat. Beide Chemikalien sind nachweisbar starke Cancerogene.

An heteroaromatische oder aromatische Nitrile kann man Ammoniak unter Druck in flüssigem Ammoniak direkt addieren, allerdings erfordert dies lange Reaktionszeiten (16 h) und liefert die Produkte nur in schlechten Ausbeuten (40 %) (P.C. Srivastava, J. Med. Chem. 27 (1984) 266). Weiter kann man aus den Nitrilen mit Hydroxylamin unter reduktiver Spaltung der intermediären Carbonsäureamidoxime Amidine synthetisieren (H. Jendralla, Tetrahedron 51 (1995) 12047). Die reduktive Spaltung schränkt jedoch das Substitutionsmuster des Nitrils erheblich ein. Doppelbindungen oder Nitrogruppen werden ebenfalls leicht hydriert. Schutzgruppen, wie beispielsweise die Benzyl-Gruppe werden ebenfalls leicht abgespalten.

1986 publizierte A. Eschenmoser eine Cystein-katalysierte Amidin-Synthese(Helv. Chim. Acta 69 (1986) 1224). Die experimentelle Überprüfung dieser Synthese zeigte jedoch, daß die Ausbeute nur ca. 58 % beträgt.

Die einfachste Synthese von Amidinen ist die direkte Addition von Ammoniak an Nitrile. Versuche mit substituierten Nitrilen zeigten jedoch, daß die Umsetzung auch unter Druck nur gering und die Ausbeute an Amidin daher entsprechend gering ist.

Der Erfindung lag die Aufgabe zugrunde, eine Methode zu entwikkeln, nach der auch Nitrile mit aufwendigen Substitutionsmustern, die einer konventionellen Amidinsynthese unzugänglich sind, auf einfache Art und Weise in die entsprechenden Amidine überführt werden können.

Gegenstand der Erfindung ist ein Verfahren zur Herstellung von Amidinen und deren Salzen mit anorganischen oder organischen Säuren, dadurch gekennzeichnet, daß man das entsprechende Nitril in Gegenwart einer Mercaptocarbonsäure, die außer der SH- und COOH-Gruppe keine weiteren unter den Reaktionsbedingungen reaktive Gruppen trägt, und gegebenenfalls in Gegenwart eines anorganischen oder organischen Ammoniumsalzes mit Ammoniak, einem C₁-C₆-Alkylamin oder Hydrazin umsetzt.

Nach dem Verfahren lassen sich praktisch alle Amidine der Formel I

R-C(NHR') = NH (I)

herstellen, in denen R einen aliphatischen, aromatischen oder heterocyclischen Rest und R' ein Wasserstoffatom, einen C₁₋₆-Alkylrest oder eine Aminogruppe darstellen.

In der Formel I kann R ein Derivat des Benzols darstellen, wie beispielsweise Phenyl, o-Fluorphenyl, m-Fluorphenyl, p-Fluorphenyl, o-Chlorphenyl, m-Chlorphenyl, p-Chlorphenyl, o-Nitrophenyl, m-Nitrophenyl, p-Nitrophenyl, o-Methoxyphenyl, m-Methoxyphenyl oder p-Methoxyphenyl.

Weiter kann R ein heterocyclisches System, insbesondere ein Derivat des Pyridins, Pyrimidins, Thiophens, Furans, Pyrrols, Isoxazols, 1,2,4-Oxadiazols, Pyrrolins oder Pyrrolidins sein wie beispielsweise Pyridin-2-yl, Pyridin-3-yl, Pyridin-4-yl, Isoxazol-3-yl, 1,2,4-oxadiazol-3-yl, Pyrimidin-2-yl, Pyrimidin-4-yl oder Thiophen-2-yl.

Von den genannten Ringen ist der Pyridin-Ring bevorzugt, besonders, wenn er in der 2-Stellung durch eine Cyano-Gruppe substituiert ist. Bevorzugt ist auch der Isoxazolring mit einer Cyanofunktion in 3-Stellung sowie der 1,2,4-Oxadiazolring mit einer Cyanofunktion in 3-Stellung.

Schließlich kann R auch eine oligopeptidische Struktur darstellen, die aus bis zu 12 natürlichen Aminosäuren, den entsprechenden D-Aminosäuren oder den natürlichen Aminosäuren sehr ähnlichen Verbindungen besteht. Speziell seien folgende Aminosäuren genannt:
Glycin, Alanin, Phenylalanin, Prolin, Valin, 2,3-, 3,4- oder 4,5-Didehydroprolin, Cyclohexylalanin.

Von ganz besonderem Interesse ist das Verfahren für die Herstellung von den in letzter Zeit bekannt gewordenen Thrombin-Inhibitoren, die einen Amidin-Rest tragen und die beispielsweise in den Patentanmeldungen WO 94/29335, WO 94/29336, WO 95/23609, EP 669.317, WO 95/35309 genannt sind. Diese besitzen überwiegend die folgende Struktur: 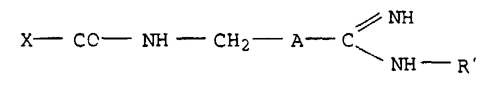 worin X der Rest einer gegebenenfalls substituierten Aminosäure, vorzugsweise Prolin oder Didehydroprolin, und
A der Rest 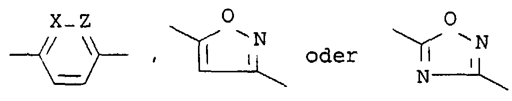 darstellt,
worin Y und Z CH- oder NH-Gruppen darstellen.

Asymmetriezentren in den Verbindungen der Formel I stören die Reaktion nicht und bleiben bei der Reaktion erhalten.

Die Umsetzung erfolgt in einem inerten Lösungsmittel, bevorzugt in Lösungsmitteln, in denen Ammoniak bei 0°C und 1 bar besser löslich ist als 2 Gew.-%. Solche Lösungsmittel sind insbesondere Alkohole, wie Methanol und Ethanol. Gleiche Überlegungen gelten für die Verwendung von Aminen und Hydrazin.

Die Reaktion führt man im allgemeinen im Temperaturbereich von -10 bis 200°C und einem Druckbereich von 1 bis 20 bar durch. Bevorzugt sind die Siedepunkttemperatur des Reaktionsgemischs und 1 bar. Ganz besonders bevorzugt ist die Durchführung der Reaktion unter Eigendruck. Wird die Reaktion ohne Druck durchgeführt, ist gelegentliches Nachsättigen mit Ammoniak oder Aminen erforderlich.

Die Reaktion kann man in Gegenwart eines Ammoniumsalzes durchführen. Man erhält dann in der Regel die entsprechenden Amidiniumsalze. Verwendet man ein Ammoniumsalz, so sollte dieses das Salz einer Säure sein, die stärker ist als die verwendete Mercaptocarbonsäure. Speziell seien als Salze die der Halogenwasserstoffsäuren (insbesondere Salzsäure), Schwefelsäure, Phosphorsäure, Salpetersäure und C₁₋₆-Carbonsäuren genannt.

Bevorzugt ist jedoch die Umsetzung in Abwesenheit eines Ammoniumsalzes. Das Reaktionsprodukt ist dann das Amidiniumsalz der Mercaptocarbonsäure. Die Mercaptocarbonsäure übt neben dem katalytischen Effekt noch eine stabilisierende Wirkung auf das Amidin aus. Die Mercaptocarbonsäure wird in der Regel in einer Menge von 0,05 bis 5 Mol, vorzugsweise etwa 1 Mol pro Mol Nitril in die Reaktion eingesetzt.

Ein besonderer Vorteil der Mercaptocarbonsäure ist, daß es sich hierbei in aller Regel um geruchlose bis kaum riechende Verbindungen handelt, während die in der Literatur beschriebenen Verfahren oft übel riechende und sehr giftige Verbindungen vorsehen.

Als Mercaptocarbonsäuren eignen sich solche, die außer der SH- und der COOH-Gruppe keine weiteren reaktive Gruppen tragen. Insbesondere kommen solche der Formel HS-R'-COOH in Betracht, worin R' für einen C₁₋₁₂-Alkylenrest stellt, wobei die Kohlenstoffkette bis zu 3 Ringe enthalten und durch unter den Reaktionsbedingungen sich inert verhaltende Heteroatome wie Stickstoff und Sauerstoff unterbrochen oder substituiert sein kann. Vorzugsweise bedeutet R' einen C₁₋₆-Alkylenrest oder eine Phenylen-Gruppe, die einfach oder zweifach durch folgende Reste substituiert sein kann: Methyl, Methoxy, Ethoxy, n-Propoxy, i-Propoxy, C₁₋₆-Alkylamino, C₁₋₆-Dialkylamino-, Halogen, Nitro.

Speziell seien Mercaptoessigsäure, α- und β-Mercaptopropionsäure, N-acylierte Aminothiocarbonsäuren - wie N-acylierte Cysteine-, Mercaptoalkylenproline - wie N-(3-Mercaptopropyl)-prolin-, Mercaptoalkanoylproline - wie N-(3-Mercaptopropionyl)-prolin-, oder cyclische Thiocarbonsäuren - wie Mercaptobenzoesäure - genannt.

Als besonders günstig haben sich für das Verfahren die Substanzen Captopril und Acetylcystein erwiesen.

In der Regel bricht man die Reaktion in üblicher Weise ab, wenn kein Nitril im Reaktionsgemische mehr nachgewiesen werden kann (z.B. mittels GC, HPLC, DC).

Die Aufarbeitung auf das Verfahrensprodukt hin erfolgt in der Regel nach herkömmlichen Verfahren, wie Destillation, Filtration, Zentrifugation oder Extraktion.

Das erfindungsgemäße Verfahren ist diskontinuierlich, z.B. in einem Rührreaktor durchführbar. Die einfache Durchführbarkeit des Verfahrens bietet den Vorteil, daß man es auf kontinuierliche Arbeitsweise, beispielsweise unter Verwendung eines Reaktionsrohres oder einer Rührreaktorkaskade, umstellen kann.

Die Stereochemie der Mercaptocarbonsäuren spielt für deren Wirkung in der beanspruchten Reaktion keine Rolle.

Die erhaltenen Rohprodukte können gewünschtenfalls weiter gereinigt werden, z.B. durch Kristallisation, Extraktion oder Chromatographie.

Überraschenderweise zeigte sich, daß bei der Durchführung des erfindungsgemäßen Verfahrens unerwünschte Nebenreaktionen unterbleiben und die Umsetzung quantitativ ist, wenn man Mercaptocarbonsäuren verwendet.

### Beispiel 1: Synthese von (S)-(3,4-Dehydroprolin-(6-amidino-3-picoiinyl)-amid mit N-Acetyl-(S)-cystein als Katalysator

2,63 g (10 mmol) (S)-(3,4-Dehydroprolin-(6-cyano-3-picolinyl)-amid-hydrochlorid wurden zusammen mit 1,79 g (11 mmol) N-Acetyl-(S)-cystein in 10 ml Methanol vorgelegt. Die Reaktionsmischung wurde bei 65°C mit Ammoniak gesättigt. Nach 2 h war mittels Dünnschichtchromatographie kein Edukt mehr nachzuweisen. Der Reaktionsansatz wurde am Rotationsverdampfer eingeengt. Es wurden 4,7 g eines fast farblosen Feststoffs erhalten, der 70,5 % des gewünschten Produktes enthielt. Fp.: 66°C, ¹³C, NMR (CDCl₃, ppm): 162,1 (Amidin).

### Beispiel 2: Synthese von N-((t-Butoxycarbonyl)-methylen)-(R)-cyclohexylalanyl-(S)-prolin-(6-amidino-3-picolinyl)-amid mit N-Acetyl-(S)-cystein als Katalysator

50 g (105 mmol) N-((t-Butoxycarbonyl)-methylen)-(R)-cyclohexylalanyl-(S)-prolin-(6-cyano-3-picolinyl)-amid wurden zusammen mit 17,7 g (109 mmol) N-Acetyl-(S)-cystein in 50 ml Methanol vorgelegt. Die Reaktionsmischung wurde bei 65°C mit Ammoniak gesättigt. Nach 4 h war mittels Dünnschichtchromatographie kein Edukt mehr nachzuweisen. Der Reaktionsansatz wurde am Rotationsverdampfer eingeengt. Es wurden 70,3 g eines fast farblosen Feststoffs erhalten, welcher 75,3 % N-((t-Butoxycarbonyl)-methylen)-(R)- cyclohexylalanyl-(S)-prolin-(6-amidino-3-picolinyl)-amid enthielt, ¹³C - NMR (CDCl₃, ppm): 162,3 (Amidin).

### Beispiel 3: Synthese von N-Boc-N-((t-Butoxycarbonyl)-methylen)-(R)-cyclohexylalanyl-(S)-(3,4-dehydroprolin-(6-amidino-3-picolinyl)-amid mit N-Acetyl-(S)-cystein als Katalysator

124,3 g (197 mmol) N-Boc-N-((t-Butoxycarbonyl)-methylen)-(R)-cyclohexylalanyl-(S)-(3,4-dehydroprolin-(6-cyano-3-picolinyl)-amid wurden zusammen mit 35,5 g (218 mmol) N-Acetyl-(S)-cystein in 400 ml Methanol vorgelegt. Die Reaktionsmischung wurde bei 65°C mit Ammoniak gesättigt. Nach 6,5 h war mittels Dünnschichtchromatographie kein Edukt mehr nachzuweisen. Der Reaktionsansatz wurde am Rotationsverdampfer eingeengt. Es wurden 165,2 g eines fast farblosen Feststoffs erhalten, der 81,6 % N-Boc-N-((t-Butoxycarbonyl)-methylen)-(R)-cyclohexylalanyl-(S)-(3,4-dehydroprolin-(6-amidino-3-picolinyl)-amid enthielt, Fp.: 91-118°C (Zersetzung). MS (ESI): 612,4 g/mol.

### Beispiel 4: Synthese von N-((t-Butoxycarbonyl)-methylen)-(R)-cyclohexylalanyl-(S)-(3,4-dehydroprolin-(6-amidino-3-picolinyl)amid mit N-Acetyl-(S)-cystein als Katalysator

2,5 g (5 mmol) N-((t-Butoxycarbonyl)-methylen)-(R)-cyclohexylalanyl-(S)-(3,4-dehydroprolin-(6-cyano-3-picolinyl)-amid wurden zusammen mit 0,89 g (5,5 mmol) N-Acetyl-(S)-cystein in 6 ml Methanol vorgelegt. Die Reaktionsmischung wurde bei 65°C mit Ammoniak gesättigt. Nach 5 h war mittels Dünnschichtchromatographie kein Edukt mehr nachzuweisen. Der Reaktionsansatz wurde am Rotationsverdampfer eingeengt. Es wurden 3,3 g eines fast farblosen Feststoffs erhalten, der 73,6 % N-((t-Butoxycarbonyl)-methylen)-(R)-cyclohexylalanyl-(S)-(3,4-dehydroprolin-(6-amidino-3-picolinyl)-amid enthielt.

### Beispiel 5: Synthese von N-((t-Butoxycarbonyl)-methylen)-(R)-cyclohexylalanyl-(S)-prolin-(6-amidino-3-picolinyl)-amid mit Mercaptoessigsäure als Katalysator

5 g (10,5 mmol) N-((t-Butoxycarbonyl)-methylen)-(R)-cyclohexylalanyl-(S)-prolin-(6-cyano-3-picolinyl)-amid wurden zusammen mit 1,1 g (12 mmol) Mercaptoessigsäure in 10 ml Methanol vorgelegt. Die Reaktionsmischung wurde bei 25°C mit Ammoniak gesättigt. Nach 4 h war mittels Dünnschichtchromatographie kein Edukt mehr nachzuweisen. Der Reaktionsansatz wurde am Rotationsverdampfer eingeengt. Es wurden 6,3 g eines grünen Feststoffs erhalten,der 70 % N-((t-Butoxycarbonyl)-methylen)-(R)-cyclohexylalanyl-(S)-prolin-(6-amidino-3-picolinyl)-amid enthielt.

### Beispiel 6: Synthese von N-Boc-N-((t-Butoxycarbonyl)-methylen)-(R)-cyclohexylalanyl-(S)-(3,4-dehydroprolin-(6-amidino-3-picolinyl)-amid mit N-((R)-3-Mercaptoisobutanoyl)-(S)-prolin (Captopril) als Katalysator

3 g (5 mmol) N-Boc-N-((t-Butoxycarbonyl)-methylen)-(R)-cyclohexylalanyl-(S)-(3,4-dehydroprolin-(6-cyano-3-picolinyl)-amid wurden zusammen mit 1,21 g (5,5 mmol) N-((R)-3-Mercaptoisobutanoyl)-(S)-prolin in 8 ml Methanol vorgelegt. Die Reaktionsmischung wurde bei 65°C mit Ammoniak gesättigt. Nach 4 h war mittels Dünnschichtchromatographie kein Edukt mehr nachzuweisen. Der Reaktionsansatz wurde am Rotationsverdampfer eingeengt. Es wurden 4,1 g eines fast farblosen Feststoffs erhalten, der 54,9 % N-Boc-N-((t-Butoxycarbonyl)-methylen)-(R)-cyclohexylalanyl-(S)-(3,4-dehydroprolin-(6-amidino-3-picolinyl)-amid enthielt, MS (ESI): 612,4 g/mol.
13C - NMR (CDCl₃, ppm): 162,1 (Amidin).

### Beispiel 7: Synthese von Boc-(R)-Cyclohexylalanyl-(S)-prolin--(6-amidino-3-picolinyl)-amid mit N-Acetyl-(S)-cystein als Katalysator

5 g (10,5 mmol) Boc-(R)-Cyclohexylalanyl-(S)-prolin-(6-cyano-3-picolinyl)-amid wurden zusammen mit 1,7 g (10,5 mmol) N-Acetyl-(S)-cystein in 20 ml Methanol vorgelegt. Die Reaktionsmischung wurde bei 65°C mit Ammoniak gesättigt. Nach 5 h war mittels Dünnschichtchromatographie kein Edukt mehr nachzuweisen. Der Reaktionsansatz wurde nach Rühren über Nacht am Rotationsverdampfer eingeengt. Es wurden 7 g eines fast farblosen Feststoffs erhalten, der 78,3 % Boc-(R)-Cyclohexylalanyl-(S)-prolin-(6-amidino-3-picolinyl)-amid enthielt, ¹³C - NMR (CDCl₃, ppm): 162,3 (Amidin).
Analog Beispiel 1 wurden folgende Verbindungen synthetisiert:

### Beispiel 8: N-(1,3-Dihydroxy-propan-2-yl)-(R)-cyclohexylglycyl-(S)-prolin-(6-amidino-3-picolinyl)amid

¹³C-NMR (DMSO, ppm): δ = 162,3 (Amidin), FAB-MS: (M + H)⁺ = 461.

### Beispiel 9: N-Boc-N-((t-Butoxycarbonyl)-ethylen)-(R)-cyclohexylalanyl-(S)-prolin-(6-amidino-3-picolinyl)amid

¹³C-NMR (DMSO, ppm): δ = 161,9 (Amidin), FAB-MS: (M + H)⁺ = 629.

### Beispiel 10: N-Boc-N-((t-Butoxycarbonyl)-methylen)-(R)-cyclohexylalanyl-1-aminocyclopropan-1-carbonsaure-(6-amidino-3-picolinyl)amid

¹³C-NMR (DMSO, ppm): δ = 162,2 (Amidin), FAB-MS: (M + H)⁺ = 601,5.

### Beispiel 11: N-(6-Amidinopyridin-3-ylmethyl)-2-(2-oxo-1-phenylmethansulfonylamino-pyrrolidin-1-yl)-acetamid

FAB-MS: (M + H)⁺ = 445.

### Beispiel 12: N[(t-Butoxycarbonyl)-methylen]-N-Boc-(R)-cyclohexylalanyl-(S)-N-methylalanin-(6-amidino-3-picolinyl)-amid

FAB-MS: (M + H)⁺ = 603.

### Beispiel 13: N[(t-Butoxycarbonyl)-methylen]-N-benzyl-glycyl-(S)-3,4-dehydroprolin-(6-amidino-3-picolinyl)-amid

FAB-MS: (M + H)⁺ = 507.

### Beispiel 14: N-Boc-N-[(Butoxycarbonyl)-methylen]-(R)-cyclohexylalanyl-(S)-(3,4-dehydroprolin-(6-(N-methyl)amidino-3-picolinyl)-amid

FAB-MS: (M + H)⁺ = 626.6.

### Beispiel 15: N-Boc-N-[(t-Butoxycarbonyl)-methylen]-(R)-cyclohexylalanyl-(S)-(3,4-dehydroprolin-(6-(N-amino)amidino-3-picolinyl)-amid

FAB-MS: (M + H)⁺ = 627.6.

### Beispiel 16: N-Boc-N[(t-Butoxycarbonyl)-methylen]-(R)-cyclohexylalanyl-(4,4-dimethyl)prolin-(6-amidino-3-picolinyl)-amid

FAB-MS: (M + H)⁺ = 642.7.

### Beispiel 17: N-Boc-N[(t-Butoxycarbonyl)-methylen]-(R)-cyclohexylalanyl-(S)-(3,4-dehydroprolin-(3-amidino-isoxazol-5-yl)-methylamid

FAB-MS: (M + H)⁺ = 602.7.

### Beispiel 18: 3-Amidino-5-N-Boc-aminomethyl-1,2,4-oxadiazol

FAB-MS: (M + H)⁺ = 242.

### Beispiel 19: 3-(2-Trifluoromethylbenzyl)-benzoyl-(5)-3,4-dehydroprolin-(6-amidino-3-picolinyl)-amid-acetat

Weiße Kristalle, Fp. 188-191°C, FAB-MS: (M + H)⁺ = 508.

### Beispiel 20: 9-Hydroxyfluorenyl-9-carboxy-(S)-3,4-dehydroprolin-(6-amidino-3-picolinyl)-amid-acetat

Weiße Kristalle, Fp. 181-185°C (Zersetzung),
FAB-MS: (M + H)⁺ = 454.

### Beispiel 21: N-Methylsulfonyl-(R)-cyclohexylalanyl-(S)-3,4-dehydroprolin-(6-amidino-3-picolinyl)-amid-acetat

Weiße Kristalle, Fp. 175-176°C, FAB-MS: (M + H)⁺ = 477.

### Vergleichsversuch mit Ammoniak ohne Katalysator

In einem 300 ml Autoklaven wurden 10 g (21 mmol) Boc-(R)-Cyclohexylalanyl-(S)-prolin-(6-cyano-3-picolinyl)-amid und 2,25 g (42 mmol) Ammoniumchlorid in 100 ml Methanol zusammen mit 60 ml flüssigem Ammoniak vorgelegt und durch Aufpressen von Stickstoff ein Innendruck von 40 bar eingestellt. Nach einer Reaktionszeit von 100 h bei 30°C wurde der Reaktionsansatz am Rotationsverdampfer einrotiert. Die Ausbeute betrug nach HPLC nur 48,5 % Boc-(R)-Cyclohexylalanyl-(S)-prolin-(6-amidino-3-picolinyl)-amid.

### Vergleichsversuch mit Cystein als Katalysator

10 g (21 mmol) Boc-(R)-Cyclohexylalanyl-(S)-prolin-(6-cyano-3-picolinyl)-amid wurden zusammen mit 2,25 g Ammoniumchlorid und 2,54 g (21 mmol) (S)-Cystein in 100 ml Methanol vorgelegt. Die Reaktionsmischung wurde bei 20-30°C mit Ammoniak gesättigt. Nach 1,5 h war mittels Dünnschichtchromatographie kein Edukt mehr nachzuweisen. Der Reaktionsansatz wurde nach Rühren über Nacht am Rotationsverdampfer eingeengt. Es wurden 15 g eines fast farblosen Feststoffs erhalten, der gemäß HPLC 40 % Boc-(R)-Cyclohexylalanyl-(S)- prolin-(6-amidino-3-picolinyl)-amid enthielt.

## Patentansprüche

1. Verfahren zur Herstellung von Amidinen und deren Salzen mit anorganischen oder organischen Säuren, dadurch gekennzeichnet, daß man das entsprechende Nitril in Gegenwart einer Mercaptocarbonsäure, die außer der SH- und COOH-Gruppe keine weiteren unter den Reaktionsbedingungen reaktive Gruppen trägt, und gegebenenfalls in Gegenwart eines anorganischen oder organischen Ammoniumsalzes mit Ammoniak, einem C₁-C₆-Alkylamin oder Hydrazin umsetzt.

2. Verfahren gemäß Anspruch 1, dadurch gekennzeichnet, daß man es in Abwesenheit eines Ammoniumsalzes durchführt.

## Claims

1. A process for preparing amidines and their salts with inorganic or organic acids, which comprises reacting the corresponding nitrile with ammonia, a C₁-C₆-alkylamine or hydrazine in the presence of a mercaptocarboxylic acid carrying, apart from the SH and the COOH groups, no other groups reactive under the reaction conditions, and in the presence or absence of an inorganic or organic ammonium salt.

2. The process as claimed in claim 1, wherein the process is carried out in the absence of an ammonium salt.

## Revendications

1. Procédé pour la préparation d'amidines et de leurs sels d'acides minéraux ou organiques, caractérisé par le fait que l'on fait réagir le nitrile correspondant avec l'ammoniac, une alkylamine en C1-C6 ou l'hydrazine en présence d'un acide mercaptocarboxylique qui, en dehors des groupes SH et COOH, ne porte pas d'autre groupe réactif dans les conditions observées, et le cas échéant en présence d'un sel d'ammonium organique ou minéral.

2. Procédé selon la revendication 1, caractérisé par le fait que l'on opère en l'absence d'un sel d'ammonium.
